# EUROPÄISCHE PATENTANMELDUNG

(11) **EP 1 669 064 A1**
(43) Veröffentlichungstag der Anmeldung: **14.06.2006**
(21) Anmeldenummer: 05025567.8
(22) Anmeldetag: 23.11.2005
(51) Int. Cl.: A61K 31/167

(54) **Zusammensetzung und Methode einer topischen Therapie von Neurodermitis**

(30) Priorität: 10.12.2004 DE 102004059613
(71) Anmelder: BIONICS PHARMA GMBH, 82031 Grünwald bei München (DE)
(72) Erfinder: Liedtke, Rainer K., Dr. c/oBIONICS PHARMA GmbH, 82031 Grünwald (DE)
(74) Vertreter: TBK-Patent

(57) **Zusammenfassung**

Die Erfindung beschreibt die Zusammensetzung und Methode und einer topischen Therapie der Neurodermitis, insbesondere derer Hautschmerz- und Reiz-Symptomatik. Dabei wird eine dermal geeignete topische pharmazeutische Formulierung eingesetzt, die in geeigneter Dosis mit einem Natrium-Kanal Blocker aus der Klasse der Lokalanästhetika vom Ester- oder Amid-Typ beladen ist, und diesen gezielt auf oder unter die Hautregion freisetzt. Durch Hemmung der, auf die initial zellulär inflammatorischen Schmerzfaktoren bewirkten, reaktiven Transmission neuronaler Schmerzimpulse erreicht dies eine pharmakologisch rasche und effektive Senkung von Hautreizung und Hautschmerz neurodermitischer Ursache. Der von der primären Beseitigung nervaler Irritationssymptomatik sich im Sinne einer retrograd verlaufenden Kettenreaktion bis hin zu morphologischen Besserungen ausbreitende Therapieeffekt zeigt dabei auch hautkurative Ansätze was durch einen assoziierten vasotropen Effekt Verbesserte lokale Durchblutung kommt dabei mutmasslich über die lokale Verschaltung afferenter Schmerzfasern mit postganglionär sympathischen Fasern in der spinalen dorsalen Wurzel zustande, stellt somit einen indirekten α-Rezeptoren blockierenden Effekt des Natrium Kanal Blockers dar.

## Beschreibung

Die Erfindung betrifft die Methode und Zusammensetzung einer topischen Therapie dermaler Reiz- und Schmerz-Symptomatik von Neurodermitis mittels pharmazeutischer Formulierungen über die Haut.

Neurodermitis ist eine meist schubweise auftretende Entzündungsreaktion der Haut mit chronischem Charakter, die zu sogenannten atopischen Erkrankungen gehört. Bei diesen produziert das körpereigene Immunsystem starke oder inadequate Abwehrreaktionen gegen Stoffe aus der Umwelt, sogenannte Allergene. Aber auch psychische Faktoren können Schübe auslösen oder diese verstärken. Der Name leitet sich aus einer Wortkombination aus Neuron (Nerv), Derma (Haut) sowie Endung -itis für Entzündung ab.
Die Neurodermitis beginnt häufig schon im Kleinkindalter. Die befallene Haut erscheint trockener als normale Haut, hat verminderte Speicherfähigkeit für Feuchtigkeit und verringert auch ihre Funktion als mechanische Barriere gegen Umweltstoffe. Zudem ist meist die Regulation weiterer Hautfunktionen wie Durchblutung, Schweißbildung und Temperatur gestört. Dadurch wird die Haut spröde und rauh, neigt zu Schuppung und ist dabei auch deutlich empfindlicher gegen chemische oder mechanische Reize. Wir sehen dabei als Ursache sekundäre Reizerscheinungen auf das periphere neuronale Schmerzleitungssystem an, was erklären kann dass sich je, nach erreichtem Ausprägungsgrad, die Hautirritation über einen breiten Bereich von leichteren Reizsymptomatiken bis hin zu sehr berührungsempfindlichem Schmerzempfinden, d.h. Schmerz schon auf solche Reize, die normalerweise nicht schmerzhaft sind (Allodynie), erstrecken kann. Dies stellt daher auch eine starke psychische Belastung dar.

Zum Begriff dermale Reiz- und Schmerz-Symptomatik sei daher dazu hier noch aufgeführt, dass damit im nachfolgenden und im Kontext stets solche Krankheitszeichen gemeint sind, die subjektive negative Empfindungen zur und von der Haut darstellen, und die dabei den Bereich von einer gemischten unangenehmen Reizempfindung bis hin zu reiner Schmerzempfindung einnehmen können.

Da man sich bisher auf keinen einheitlichen Namen für die Krankheit einigen konnte, bestehen zu ihr noch zahlreiche synonyme Bezeichnungen, unter anderem, atopische Dermatitis, atopisches Ekzem, endogenes Ekzem, Neurodennitis atopica. Der synonym häufig genutzte Begriff "atopische Dermatitis" soll ausdrücken, dass die Erkrankungsschübe auch ohne erkennbare äußere Ursache, auftreten können. "Allergische Dermatosen" bezeichnet dabei summarisch die bei Allergien hervorgerufenen Hautveränderungen. Im nachfolgenden und dessen Kontext sind daher mit dem als funktionell übergeordnet angesehen Begriff Neurodermitis stets auch diese synonym eingestetzten Bezeichnungen gemeint und mit eingeschlossen.

Nach derzeitiger Sicht ist die primäre Ursache der Neurodermitis eine überschießende Abwehrreaktion des Körpers gegen exogene Stoffe z.B. aus Nahrungsmitteln. Auslösend oder verschlechternd können aber auch mechanische Reizungen der Haut, Infektionen oder klimatische Bedingungen sein. Dabei richten sich spezielle Untergruppen der weißen Blutkörperchen, Lymphozyten, gegen diese Allergene. Dies fuhrt zur Bildung spezieller Abwehrstoffe, Antikörper. Im Zusammenspiel mit verschiedenen Botenstoffen des Immunsystems, sogenannten Cytokinen, kommt dann es zu einer entzündlichen Abwehrreaktion der Haut. Diese wird durch verschiedene weisse Blukörperchen (Lymphozyten), weiter in Gang gehalten. Hierbei werden in der Haut u.a. auch entzündungsfördernde Stoffe ausgeschüttet, was die Abwehrreaktion und deren Hauptsymptome weiter verstärkt.

Infolge ihrer Alltagsursachen sind Prevalenz und Inzidenz in der Gesamtbevölkerung hoch. Die Neurodermitis gehört daher zu den häufigsten Hauterkrankungen, wobei Kinder überdurchschnittlich betroffen sind.

Pharmakologisch existiert derzeit keine solche Therapie die man als einen allgemeingültigen Standard ansehen kann, sondern erfolgt in der Regel mehr empirisch und nach den individuellen Gegebenheiten. Reizende und schmerzhafte Hautsymptomatiken der Neurodermitis werden sehr oft zunächst mit topischen pharmazeutischen Formulierungen angegangen die Cortison enthalten, ansonsten unter anderem mit Formulierungen die beispielweise als Ingredientien Zink, bestimmte Öle, Antihistaminika oder auch pflanzliche Bestandteile enthalten können, neuerdings auch zunehmend sogenannte Immunsupressiva in Salbenform. In vielen Fällen, vor allem Langzeitfällen, handelt es sich nur um wirkstoffreie

Salben oder Gele, die eine Minderung des Reizustandes der Haut herbeiführen sollen. Bei Verdacht auf infektiöse Ursachen oder deren Mitbeteiligung erfolgt zudem die Gabe topischer oder systemischer Antibiotika. Eine Heilung der Erkrankung durch eine ursächliche (kausale) Therapie erscheint derzeit noch nicht möglich. Insgesamt sind die derzeitigen symptomatischen Therapien daher meist langwierig, teils mit geringen Ansprechraten und in vielen individuellen Fällen auch nicht erfolgreich. Zudem beinhalten systemische Therapien, z.B. mit Cortison, signifikante Nebenwirkungen. Daher existieren in diesem Bereich auch zahlreiche Therapieversuche mit sogenannten Alternativ-Therapien.

Zur Art der Verabreichung sei hier noch vorab der Begriff "topische Anwendung" als Gegensatz zur systemischen Anwendung von Arzneimitteln dargestellt. Mit einer topischen Anwendung ist nachfolgend gemeint die lokale Verabreichung von Arzneistoffen. Im Bereich einer Therapie von Reizungerscheinungen und Schmerz der Haut ist dies daher die Verabreichung des Arzneistoffes direkt auf eine gereizte oder schmerzhafte Hautregion. Dies kann beispielweise mit einer Salbe oder einem Gel der Fall sein. Der Wirkort ist dabei somit weitgehend identisch mit dem Verabreichungsort. Dies steht im Gegensatz zu einer systemischen Verabreichung, bei der ein Arzneistoff, beispielsweise mit einer Tablette, nach der Aufnahme über Magen und Darm erst über die Blutbahn im gesamten Körper verteilt wird und erst hiernach und nur zu einem Bruchteil auch an den Ort der schmerzhaften Ursache gelangt. Dementsprechend sind bei systemischer Anwendung Verabreichungsort und Wirkort nicht identisch. Systemischen Therapien ist daher eine höhere Rate an unerwünschten Wirkungen gemeinsam. Die hier eingesetzten Begriffe wie "topische Anwendungsform" oder "topische Zusammensetzung" meinen daher pharmazeutische Zubereitungen, die für den Zweck einer topischen Anwendung erstellt sind.

Innerhalb der komplexen Gesamtkasakade des zellulären Schmerzmechanismus spielen die biochemisch geweblichen Entzündungsvorgänge als Folge allergischer Reaktionen eine wesentliche Rolle, inbesondere die Bildung von Prostaglandinen. Dies sind hormonähnliche Substanzen, die in chemisch verschiedenen Formen verbreitet im menschlichen Körper vorkommen und insgesamt eine Rolle bei der Entstehung von Entzündungsprozessen spielen, So steigern diese im Rahmen von Entzündungen u.a. die Empfindlichkeit von Schmerzrezeptoren und verstärken die Freisetzung verschiedener lokal algetisch wirkender Mediatoren und lokaler Ödembildungen.

Allgemein werden Lokalanästhetika in der Medizin als wirksame örtliche Betäubungsmittel eingesetzt, z.B bei chirurgischen Operationen, ausserdem auch einige Stoffe aus dieser Klasse, systemisch, zur Stabilisierung des Herzrhythmus. In der Schmerztherapie haben sich bei einigen Anwendungsgebieten auch lokale Injektionen mit Lokalanästhetika als klinisch erfolgreich erwiesen. Diesen lag als Basiskonzept die Ausschaltung von Nervenbahnen durch deren Betäubung zugrunde. Als ein hierbei weiter differenziertes pharmakologisches Prinzip hat sich eine nicht-invasive Anwendung von Lokalanästhetika mit topischen Pflastersystemen bei neuromuskulären Schmerzen erwiesen. Diese neue Therapie wurde erst von uns in den letzten Jahren erfunden und eingeführt und erfolgte mit US 5,776,952, für den Bereich einer topischen Anwendung von Lokalanästhetika bei Rückenschmerzen, sowie mit US 5,840,755 für Kopfschmerz. Hierbei zeigte sich auch klinisch, daß Lokalanästhetika nicht nur konventionell als Betäubungsmittel einsetzbar sind, sondern, bei neuromuskulären Schmerzen, als spezifisch neural wirkende Analgetika ansehbar sind.

Lokalanästhetika vom Amid- und Ester-Typ, z.B. Lidocain vom Amid-Typ, zeigen als pharmakologischen Wirkungsmechanismus eine Hemmung des schnellen NatriumIonen-Influx in Nervenfasern, d.h. sie wirken als sogenannte Natrium-Kanal Blocker. Über diesen spezifischen Effekt blockieren sie die Impulsleitung in Nervenfasern, was prinzipiell erst einmal alle regional vorhandenen Nervenfasem betrifft. Da jedoch die sensorischen schmerzleitenden Fasern (unmyelisierte C-Fasern sowie myelinisierte Aδ-Fasern) anatomisch dünner sind als die motorischen. Fasern (e.g. Strichartz, G. R. (Edit.): Local Anesthetics, Handbook of Experimental Pharmacology, Vol 81. Springer, Berlin-New York 1987), lassen sich hieraus, über die topisch verabreichte Dosis, auch unterschiedliche Wirkeffekte hervorrufen. In diesem Zusammenhang erscheint auch von therapeutischem Belang, dass die peripheren B- Fibern des autonomen Nervensystems in ihrer Dicke zwischen denen der C- und Aδ- Fasern liegen. Eine zusätzliche Annahme für einen dosissparenden transcutanen Therapieansatz war es, afferente bioelektrische Schmerzsignale als irreguläre neuronale Informationsmuster anzusehen, die schon über geringere Dosen an Lokalanästhetika wieder regularisiert werden können. Auch diese Voraussagen und impliziten Annahmen u.a. von US 5,776,952, US 5,840,755 einer Beeinflussung ektopischer Impulse durch Lidocain wurden dann hiernach in den letzten Jahren experimentell weiter verifiziert (Khodorova A., Meissner K., Leeson S., Strichartz G.R.: Muscle Nerve. 24; 634 (2001), Persaud N., Strichartz G.R. Pain. 99, 333(2002).

Eine peripher topisch transcutane Schmerzsenkung erfordert daher geringere Dosen an Lokalanästhetika, als solche mit denen eine Anästhesie der Nervenfunktion erzeugt wird. Daher unterscheidet sich eine transcutane Analgesie auch durch ihre Selektivität auf Schmerzrezeptoren von einer transcutanen Anästhesie, da letztere alle lokalen Rezeptoren beeeinflusst, somit auch eine Inaktivierung motorischer Funktionen herbeiführt, was im Falle des Therapieziels einer reinen Analgesie unerwünscht ist.

Insgesamt ergibt sich somit, daß Natrium-Kanal Blocker vom Typ der Lokalanästhetika in ihrem dermal reiz- und schmerzsenkenden Wirkungsmechanismus zellulär spezifische Ausschnitte der peripher lokalen Schmerzkaskade abdecken. Sie zeigen ihre für eine Schmerzsenkung relevanten spezifischen Effekte erst auf der Ebene der sich auf dem Boden einer Enzündung reaktiv, d.h. sekundär, ausbildenden neuronalen Prozesse der Weiterleitung der Schmerzsignale. Daraus lässt sich, in Kontext mit den zellulären Abläufen der Hautenzündung bei einer Neurodermitis, die im wesentlichen auch wieder die unmyelisierten C-Fasern sowie Aδ-Fasern beeinftusst, die Folgerung ableiten, dass sich bei einer topischen Anwendung eines Natrium-Kanal Blockers aus der Klasse der Lokalanästhetika dortige Reiz- und Schmerz-Symptomatiken rasch beeinflussen lassen. Therapeutisch sollte eine lokale Verabreichung dieser Stoffe daher zu einer zudem raschen einsetzenden Wirkung auf Hautreizung und Hautschmerz und den dazu verwandten oder damit assoziierten Symptomen führen. Zudem kann bei einer Normalisierung lokaler neuronaler Irritation, als Folge auch von einer lokalen Normalisierung damit vernetzter Regelmechanismen der Hautfunktionen wie Durchblutung, Schweißbildung und Temperatur ausgegangen werden. Diese Funktionsstörungen führen ja ihrerseits auch zu trophisch- morphologischen Störungen und unterhalten damit die Hautreizung und den Hautschmerz noch weiter.

Der Erfindung liegt die Aufgabe zugrunde, die dermale Reiz- und Schmerz-Symptomatik von Neurodermitis mit einer topischen Therapie über die Haut zu bessern.

Diese Aufgabe wird dadurch gelöst, daß eine für die Haut geeignete topische pharmazeutische Formulierung eingesetzt wird, die eine therapeutisch geeignete Dosis eines Natrium-Kanal Blockers aus der Klasse der Lokalanästhetika vom Ester- oder Amid-Typ enthält, welche den enthaltenen Stoffe gezielt auf oder in die unter der pharmazeutischen Formulierung liegende Hautregion freisetzt.

Um die Anwendung der Therapie zu verbreitern, ist in einer weiteren Ausbildung der Erfindung die topisch pharmazeutische Formulierung eine Salbe, ein Gel, eine Emulsion, Suspension oder Lotion, eine Lösung oder ein Spray.

Um eine geeignete Therapie zu spezifizieren ist in der topisch pharmazeutischen Formulierung als Wirkstoff Lidocain, als Natrium-Kanal Blocker aus der Klasse der Lokalanästhetika vom Amid-Typ, eingesetzt, wobei dieser Stoff in einer Konzentration von 0,1% - 20% vorliegen kann.

Um die Wirksamkeit und Verträglichkeit der Therapie zu verbessern ist in der topisch pharmazeutischen Formulierung als weiterer Natrium-Kanal Blocker aus der Klasse der Lokalanästhetika vom Amid- oder Ester-Typ eingesetzt, Tetracain, Prilocain, Bupivacain, Mepivacain, Etidocain, Procain, Benzocain, Propoxycain, Hydroxyprocain, Chloroprocain, Ambucain, Metabutoxycain, Proparacain, Paraethoxycain, Butacain, Isobucain, Hexylcain, Piridocain, Piperocain, Cyclomethycain, Procainamid, Dibucain, Pyrrocain oder Tolycain, wobei der Stoff in einer Konzentration von 0,1% - 20% vorliegen kann.

Um die medizinische Anwendung der Therapie zu verbreitern wird die topisch pharmazeutische Formulierung eingesetzt zur Behandlung schmerzhafter oder durch Reizung bedingter Hautsymptome, die auf Basis primärer oder sekundärer lokaler Nervenirritation auftreten, einschliesslich der Symptome wie sie bei den Hauterkrankungen Psoriasis, Rosacea, perioraler Dermatitis, Lichen ruber, Ulcus cruris und allergischen Dermatosen aufterten.

Um die medizinische Anwendung der Therapie zu verbreitern wird die topisch pharmazeutische Formulierung medizinisch im Humanbereich wie auch im Veterinärbereich eingesetzt

Eine Therapie der Neurodermitits, insbesondere derer dermale Reiz- und Schmerz-Symptomatik, mittels topisch pharmazeutischer Formulierungen, die einen Natrium-Kanal Blocker aus der Klasse der Lokalanästhetika vom Ester- oder Amid-Typ enthalten, wurde bisher medizinisch weder beschrieben noch eingesetzt. Dies erscheint nicht überraschend, da hierzu sowohl deren pharmakologisches Wirkungsprofil wie auch deren bisherige medizinisch- praktische Anwendung weit entfernt liegen. Ohne

Analyse der zellulär sequentiellen Abläufe zum lokalen Schmerzgeschehen bei Neurodermitis, einschliesslich der dort ausgelösten entzündlichen Vorgänge sowie deren hierraus resultierenden trophischen und morphologischen Folgen, wird der Nutzen dieser Stoffklasse für Neurodermitis nicht erkennbar oder offensichtlich. Erst diese Analyse zeigt, daß eine topische Therapie mit Natrium-Kanal Blockern eine Senkung von Reiz und Schmerz über eine Regulation der reaktiv irritierten sensorisch neuronalen Leitung der Schmerztransmission bewirkt. So werden die subjektiven Hauptsymptome der Neurodermitis, Hautreizung und Hautschmerz, erst in Reaktion auf die Hautentzündung durch polymodale neuronale Nozizeptoren, inbesondere unmyelisierte C-Fasern sowie Aδ-Fasern und verursacht. Auch viele in die Entzündung involvierte chemische Substanzen z.B. biogene Amine, Histamin, Serotonin, sowie Proteasen und Neuropeptide, können die C-Fasern indirekt oder direkt, aktivieren und so zu Reizung mit nervaler Irritation führen. Mechanische Einflüsse können diese sensorischen Reizungen noch verstärken. Deren Beeinflussung ist durch Natrium-Kanal Blocker möglich und sie führt dann zu einer raschen Senkung der dermalen Reiz- und Schmerz-Symtomatik. Über diesen primären pharmakologischen Effekt auf die neuronale Transmission kommt es dann aber auch, überwiegend sekundär, zu einer Regulation damit assozüerter lokaler vegetativer Funktionen der Haut, die von Durchblutung, Schweißbildung und Temperatur. Hierzu nehmen wir einen, indirekten, α-Rezeptoren blockierenden Effekt der Natrium Kanal Blocker auf die Hautgefässe an, der dabei über die lokale Verschaltung der afferenten C- und Aδ- Fibern mit den postganglionär sympathischen B-Fasern in der spinalen dorsalen Wurzel zustande kommt. Solch vasotroper Effekt, mit Folge verbesserter lokaler Durchblutung, kann dann eine weitere Normalisierung trophischer Störungen und deren sichtbarer morphologischer Folgen der Haut bewirken. Dazu kann es aber auch noch, dosisabhängig, zu direkten Effekten der Natrium-Kanal Blocker an den peripheren Aα-Fibern der Blutgefässe der Haut kommen. Diese Effekte der Natrium Kanal Blocker Blocker betreffen jedoch nur die lokalen Reaktionen, da solche die über die zweite vegetative Reaktionsroute, d.h. jene die zentral hormonell über die Hyopthalamus-Nebennieren Stress Achse ausgelöst werden, hiervon nicht betroffen sind.

Der Gesamteffekt ist auch nicht über eine systemische Verabreichungen dieser Stoffe möglich. Er lässt sich nur dann therapeutisch ausreichend darstellen, wenn die Intervention, in geeigneter Dosierung, direkt am Ort der reiz- und schmerzinduzierenden Läsion erfolgt, was bei einer systemischen Dosierung nicht genügend steuerbar ist. Bei einer systemischen Anwendung bestünde zudem ein hohes Risiko unspezifischer Schäden. Anders als bei der topisch sehr sicheren Anwendung wäre beispielsweise die systemische Verabreichung eines Lokalanästhetikums ein hohes toxisches Risiko, u.a. über dessen cardiovaskuläre Effekte.

Insgesamt ergibt sich somit als Vorteil eine verbesserte und raschere Senkung der klinischen Gesamtsymptomatik, inbesondere der für den Patienten im Vordergrund stehenden dermalen Reiz- und Schmerz-Symptomatik. Dies erfolgt durch Regularisierung der durch die Entzündung irritierten terminalen Hautnerven, aus der sich aber auch sekundär noch eine Besserung des trophischen und morphologischen Hautzustandes ergibt. Dieser Therapieeffekt, der sich von der primären Beseitigung einer nervalen Irriationssymptomatik, im Sinne einer retrograd verlaufenden Kettenreaktion, bis hin zu morphologischen Besserungen ausbreitet, zeigt somit sogar Ansätze einer hautkurativen Wirkung. Dies ist derzeit mit keiner anderen bekannten Therapie, insbesondere nicht in dieser Kurzfristigkeit, erreichbar.

Beispiel 1. Ein allgemeines Anschauungsbeispiel der Ausgestaltung einer topischen pharmazeutischen Formulierung, ohne es jedoch hierauf begrenzen zu wollen, kann eine dem pharmazeutischen Fachmann vertraute Gel-Formulierung sein, bei der in 1 Gramm dieses Gels als Natrium-Kanal Blocker vom lokalanesthetischen Amid-Typus 20 Milligramm Lidocain, als Hydrochlorid, enthalten ist. Es handelt sich somit um ein Hautgel mit 2% des Wirkstoffes. Von diesem Gel kann jeweils, flächenabhängig, ca. 0,5-2 Gramm, bedarfsweise bis 3 mal täglich, dünn auf das neurodermitisch gereizte oder schmerzhafte Hautareal aufgetragen werden. Im weiteren Verlauf kann diese Dosis ausschleichend reduziert werden und braucht nur noch auf die verbleibenden gereizten Areale bedarfsmässig aufgetupft zu werden.

Die Feststellung rascher Wirksamkeit fand sich schon bei den ersten einzelnen medizinischen Fallbeobachtungen, bei denen mit einer topischen Formulierung, entsprechend Beispiel 1, bei bis dahin trotz vorheriger indifferenter Salbenbehandlung, eine schon länger anhaltende reizende und schmerzhafte Hautsymptomatik von Gesichtspartien vorlag, dann diese quälende Symtomatik innerhalb nur weniger Minuten eine signifikante Besserung zeigte. Weiterhin reduzierten sich die mikropapulösen Erscheinungen schon innerhalb der nächsten Stunden und im weiteren Verlauf kam es zu einem Abblassen der entzündlich geröteten Hautareale, mit Glättung der Hautoberfläche und sichtbarer Normalisierung der Hautdurchblutung. Weitere therapeutische Massnahmen waren nicht erforderlich.

Die Erfindung beschreibt die Zusammensetzung und Methode und einer topischen Therapie der Neurodermitis, insbesondere derer Hautschmerz- und Reiz-Symptomatik. Dabei wird eine dermal geeignete topische pharmazeutische Formulierung eingesetzt, die in geeigneter Dosis mit einem Natrium-Kanal Blocker aus der Klasse der Lokalanästhetika vom Ester- oder Amid-Typ beladen ist, und diesen gezielt auf oder unter die Hautregion freisetzt. Durch Hemmung der, auf die initial zellulär inflammatorischen Schmerzfaktoren bewirkten, reaktiven Transmission neuronaler Schmerzimpulse erreicht dies eine pharmakologisch rasche und effektive Senkung von Hautreizung und Hautschmerz neurodermitischer Ursache. Der von der primären Beseitigung nervaler Irritationssymptomatik sich im Sinne einer retrograd verlaufenden Kettenreaktion bis hin zu morphologischen Besserungen ausbreitende Therapieeffekt zeigt dabei auch hautkurative Ansätze was durch einen assoziierten vasotropen Effekt Verbesserte lokale Durchblutung kommt dabei mutmasslich über die lokale Verschaltung afferenter Schmerzfasern mit postganglionär sympathischen Fasern in der spinalen dorsalen Wurzel zustande, stellt somit einen indirekten α-Rezeptoren blockierenden Effekt des Natrium Kanal Blockers dar.

## Patentansprüche

1. Methode und Zusammensetzung einer topischen Therapie der dermalen Reiz- und Schmerz-Symptomatik von Neurodermitis, **dadurch gekennzeichnet, daß** eine für die Haut geeignete topische pharmazeutische Formulierung eingesetzt wird, welche eine therapeutisch geeignete Dosis eines Natrium-Kanal Blockers aus der Klasse der Lokalanästhetika vom Ester- oder Amid-Typ enthält, welche den enthaltenen Stoff gezielt auf oder in die unter der pharmazeutischen Formulierung liegende Hautregion freisetzt.

2. Methode und Zusammensetzung nach vorhergehendem Anspruch, **dadurch gekennzeichnet, dass** die topisch pharmazeutische Formulierung eine Salbe, ein Gel, eine Emulsion, Suspension oder Lotion, eine Lösung oder ein Spray ist.

3. Methode und Zusammensetzung nach vorhergehenden Ansprüchen, **dadurch gekennzeichnet, daß** als Wirkstoff Lidocain, als Natrium-Kanal Blocker aus der Klasse der Lokalanästhetika vom Amid-Typ, eingesetzt ist, wobei dieser Stoff in einer Konzentration von 0,1 % - 20% vorliegen kann.

4. Methode und Zusammensetzung nach vorhergehenden Ansprüchen, **dadurch gekennzeichnet, daß** als weiterer Natrium-Kanal Blocker aus der Klasse der Lokalanästhetika vom Amid- oder Ester-Typ eingesetzt ist, Tetracain, Prilocain, Bupivacain, Mepivacain, Etidocain, Procain, Benzocain, Propoxycain, Hydroxyprocain, Chloroprocain, Ambucain, Metabutoxycain, Proparacain, Paraethoxycain, Butacain, Isobucain, Hexylcain, Piridocain, Piperocain, Cyclomethycain, Procainamid, Dibucain, Pyrrocain oder Tolycain, wobei der Stoff in einer Konzentration von 0,1% - 20% vorliegen kann.

5. Methode und Zusammensetzung nach vorhergehenden Ansprüchen, **dadurch gekennzeichnet, daß** die topisch pharmazeutische Formulierung auch eingesetzt wird zur Behandlung reizender oder schmerzhafter Hautsymptome, die auf Basis primärer oder sekundärer lokaler Hautnervenirritation auftreten, einschliesslich der Symptome der Hauterkrankungen Psoriasis, Rosacea, perioraler Dermatitis Lichen ruber, Ulcus cruris und allergischer Dermatosen.

6. Methode und Zusammensetzung nach vorhergehenden Ansprüchen, **dadurch gekennzeichnet, daß** die topisch pharmazeutische Formulierung medizinisch im Humanbereich wie auch im Veterinärbereich eingesetzt wird.
